# EUROPEAN PATENT SPECIFICATION

(11) **EP 2 588 592 B1**
(45) Date of publication and mention of the grant of the patent: **06.09.2017**
(21) Application number: 11803935.3
(22) Date of filing: 30.06.2011
(51) Int. Cl.: C12N 5/00

(54) **USE OF PERFUSION DECELLULARIZED ORGANS FOR MATCHED RECELLULARIZATION**
VERWENDUNG VON PERFUSIONS-DEZELLULARISIERTEN ORGANEN FÜR ABGEGLICHENE REZELLULARISIERUNG
UTILISATION D'ORGANES DÉCELLULARISÉS PAR PERFUSION DANS LA RECELLULARISATION ADAPTÉE

(30) Priority: 30.06.2010 US 360196 P
(43) Date of publication of application: 08.05.2013
(73) Proprietor: Miromatrix Medical Inc., Eden Prairie, Minnesota 55347 (US)
(72) Inventor: ROSS, Jeffrey, Chaska, Minnesota 55318 (US)
(74) Representative: Uexküll & Stolberg
(86) International application number: PCT/US2011/001163
(87) International publication number: WO 2012/005760

(56) References cited:
- WO-A2-2010/120539
- US-A1- 2009 180 965
- US-A1- 2009 202 977
- US-A1- 2010 093 066
- E. A. ROSS ET AL: "Embryonic Stem Cells Proliferate and Differentiate when Seeded into Kidney Scaffolds", JOURNAL OF THE AMERICAN SOCIETY OF NEPHROLOGY, vol. 20, no. 11, 1 November 2009 (2009-11-01), pages 2338-2347, XP055074894, ISSN: 1046-6673, DOI: 10.1681/ASN.2008111196
- OTT H C ET AL: "Perfusion-decellularized matrix: using nature's platform to engineer a bioartificial heart", NATURE MEDICINE, NATURE PUBLISHING GROUP, NEW YORK, NY, US, vol. 14, no. 2, 1 February 2008 (2008-02-01), pages 213-221, XP002612651, ISSN: 1078-8956, DOI: 10.1038/NM1684 [retrieved on 2008-01-13]
- BASAK E UYGUN ET AL: "Organ reengineering through development of a transplantable recellularized liver graft using decellularized liver matrix", NATURE MEDICINE, vol. 16, no. 7, 13 June 2010 (2010-06-13), pages 814-820, XP055069907, ISSN: 1078-8956, DOI: 10.1038/nm.2170
- TAYLOR ET AL: "From stem cells and cadaveric matrix to engineered organs", CURRENT OPINION IN BIOTECHNOLOGY, LONDON, GB, vol. 20, no. 5, 1 October 2009 (2009-10-01), pages 598-605, XP026785527, ISSN: 0958-1669, DOI: 10.1016/J.COPBIO.2009.10.016 [retrieved on 2009-11-13]
- UYGUN ET AL.: 'Organ reengineering through development of a transplantable recellularized liver graft using decellularized liver matrix.' NATURE MEDICINE vol. 16, no. 7, 13 June 2010, pages 814 - 820, XP055069907
- KROON ET AL.: 'Pancreatic endoderm derived from human embryonic stem cells generates glucose-reresponsive insulin-secreting cells in vivo.' NATURE BIOTECHNOLOGY vol. 26, no. 4, April 2008, pages 443 - 452, XP002561975

## Description

### Background

Tissue engineering is a rapidly growing field that seeks to repair or regenerate damaged or diseased tissues and organs through the implantation of combinations of cells, scaffolds and soluble mediators. Current stem cell differentiation and primary cell culture is generally achieved under 2-dimentional (2D) culture conditions. That system allows for the expansion of specific cell populations but is limited in its ability retain functional cellular phenotypes, to support high density cell culture and long term primary or differentiated cell function. For example, in contrast to the limited availability of large numbers of primary cells needed for certain cellular therapies, the number of stem cells can be greatly expanded while retaining the ability to differentiate into specific lineages. The control of stem cell fate (e.g., differentiation), either *in vivo* or *in vitro,* has been attributed principally to genetic and molecular mediators (e.g., growth factors and transcription factors). Although stem and progenitor cell differentiation can result in cells with appropriate lineage- or tissue-specific gene expression, the differentiated cells can lack functional properties needed for *in vitro* or *in vivo* applications.

### Summary of the Invention

The invention provides perfusion decellularized organ- or tissue-derived extracellular matrix (ECM) and systems useful to support organ- or tissue-specific cellular differentiation or maturation of stem or progenitor cells, or maintenance of differentiated or primary cells, or any combination thereof, in the matrices. Primary cells are cells obtained from an organism that generally are then cultured *in vitro,* although those cells do not proliferate indefinitely. Differentiated cells include primary cells and cells that have been differentiated *in vitro,* e.g., stem cells or progenitor cells in a perfusion decellularized matrix of the invention. In one embodiment, at least 5%, 10% or 20%, or more, of the differentiated cells have a functionally mature phenotype. A tissue is a group of cells with a common structure and function, e.g., epithelial tissue, connective tissue, muscle tissue (skeletal, cardiac, or smooth muscle), and nervous tissue, and includes a pliable sheet that covers or lines or connects organs. An organ is a collection of tissues (two or more) joined in structural unit to serve a common function. Organs include but are not limited to the brain, liver, pancreas, heart, stomach, kidney, lungs, whole muscles, thymus, anus, and intestine. As used herein, an organ includes perfusable whole organs, or parts of an organ, or vascularized structures thereof, and a tissue includes any structures that contain vascularized tissues, e.g., a trachea.

In one embodiment, the present invention provides for the use of a 3D organ- or tissue-specific ECM scaffold, e.g., for matched organ- or tissue-specific differentiation or maturation, or maintenance, of cell types including stem or progenitor cells, or differentiated or primary cells. Differentiation is a process by which cells acquire a new phenotype that is distinct from the original cell population, e.g., distinct cellular gene and/or protein expression and/or function(s). Maturation further clarifies the phenotype of the cell population as having the normal mature functional capacity of a cell in an *in vivo* cell population. In one embodiment, the scaffold is a perfusion decellularized ECM sheet, e.g., a portion of an organ ECM. In another embodiment, the scaffold is a perfusion decellularized ECM organ. Such scaffolds may be employed as 3D *in vitro* culture systems, e.g., useful to provide substrates for bioreactors, in drug testing or engineered organs or tissue or cells for therapy.

Perfusion decellularized ECM from organs or tissues retains more of the native microstructure, including an intact vascular and/or microvascular system, compared to other decellularization techniques such as immersion based decellularization. For example, perfusion decellularized ECM from organs or tissues preserves the collagen content and other binding and signaling factors and vasculature structure, thus providing for a niche environment with native cues for functional differentiation or maintenance of cellular function of introduced cells. In one embodiment, perfusion decellularized ECM from organs or tissues is perfused with cells and/or media using the vasculature of the perfusion decellularized ECM under appropriate conditions, including appropriate pressure and flow to mimic the conditions normally found in the organism. The normal pressures of human sized organs is between about 40 to about 200 mm Hg with the resulting flow rate dependent upon the incoming perfusion vessel diameter. For a normal human heart the resulting perfusion flow is about 20 to about 200 mL/min/100 g. Using such a system, the seeded cells can achieve a greater seeding concentration of about 5x up to about 1000x greater than achieved under 2D cell culture conditions and, unlike a 2D culture system, the ECM environment allows for the further functional differentiation of cells, e.g., differentiation of progenitor cells into cells that demonstrate organ- or tissue-specific phenotypes having sustained function. In one embodiment, the combination of culture conditions and source of ECM allows for the functional differentiation of cells introduced to the ECM.

In one embodiment, the invention provides a method to prepare a perfusion based *in vitro* 3D cell culture having organ- or tissue-specific decellularized matrix and cells that differentiate into organ- or tissue-specific functional cell types. The method includes selecting a perfusion decellularized matrix of an organ or tissue and a population of cells including partially differentiated progenitor cells capable of differentiation to cell types present in a native organ or tissue corresponding to the decellularized organ or tissue. The selected perfusion decellularized matrix is contacted with the population of partially differentiated progenitor cells under conditions and for a period of time that provide for recellularization of the perfusion decellularized matrix and differentiation of cells in the population into functional cells. In one embodiment, the organ is a heart. In another embodiment, the organ is a liver. In another embodiment, the organ is a pancreas. In another embodiment, the organ is a lung.

In another embodiment, the invention provides a method to prepare a perfusion based *in vitro* 3D cell culture having organ- or tissue-specific decellularized matrix and cells that differentiate into organ- or tissue-specific functional cell types in which a perfusion decellularized matrix of an organ or tissue and a population of cells including differentiated cells corresponding to those present in a native organ or tissue are selected. The selected perfusion decellularized matrix is contacted with the selected population of differentiated cells under conditions and for a period of time that provide for recellularization of the perfusion decellularized matrix and functional cells.

In one embodiment, the invention provides a method to prepare a perfusion based *in vitro* 3D cell culture having organ- or tissue-specific decellularized matrix and cells that differentiate into organ- or tissue-specific functional cell types. The method includes selecting a perfusion decellularized matrix of an organ or tissue and a population of stem cells capable of differentiation to cell types present in a native organ or tissue corresponding to the decellularized organ or tissue. The selected perfusion decellularized matrix is contacted with the population of stem cells under conditions and for a period of time that provide for recellularization of the perfusion decellularized matrix and differentiation of the cells in the population into functional cells. In one embodiment, the stem cells are induced pluipotent stem (iPS) cells. In one embodiment, the stem cells are embryonic stem (ES) cells, e.g., human ES cells. In one embodiment, the stem cells are adult stem cells.

In one embodiment, a portion of an organ or tissue ECM is employed in the methods of the invention, e.g., an atrium or ventricle of a heart or interior structure of a pancreas including islets. In one embodiment, the portion is about 1 to about 2 mm in thickness. In one embodiment, the portion is about 5 to about 10 mm in thickness. In one embodiment, the portion is about 70 to about 100 mm in thickness.

In one embodiment, the invention provides for the use of perfusion decellularized ECM from a particular source (e.g., organ or tissue) for *in vitro* cell culture with cells to repopulate the ECM scaffold, where the cells, culture conditions and ECM scaffold are selected to result tissue- or organ-specific functional cell types in the ECM. In one embodiment, perfusion decellularized matrices from a particular organ or tissue allow for the culturing of introduced stem cells or partially differentiated progenitor cells expressing early lineage specific markers corresponding to the embryology of the specific organ or tissue (e.g., ES cells partially differentiated to Defined Endoderm via the expression of Sox17, Ceberus, FoxA2, and CXCR4 for seeding a pancreas scaffold) so as to yield matrices with functional, differentiated cells matched to the intended organ or tissue scaffold.

In one embodiment, partially differentiated cardiac ES or iPS cells are cultured on a perfusion decellularized cardiac ECM scaffold for a time, e.g., about 2 to about 80 days, and under conditions so as to yield cardiac cells, e.g., a substantially pure population such as one having greater than about 50% atrial cardiomyocytes when cultured on atrial cardiac ECM, greater than about 50% ventricular cardiomycytes when cultured on ventricular ECM, and/or greater than about 50% nodal cardiomyocytes when cultured on nodal ECM, which cardiomycytes are identified by their functional action potential shapes and/or action potential durations, e.g., an atrial action potential diplaying typical triangle-like action potentials compared to the the prominent plateau displayed by ventricular cardiomyocytes. This classification is based on the properties of the action potential as measured by the maximum rate of rise of the action potential (dV/dtmax), the action potential duration (APD), action potential amplitude (APA), and prominence of phase-4 depolarization. Nodal-like action potentials were characterized by prominent phase-4 depolarization, slow upstroke (dV/dtmax), and a smaller APA. Ventricular action potentials may be distinguished by the presence of a significant plateau phase of the action potential resulting in a significantly longer duration compared to the more triangular shaped embryonic-atrial action potentials. These electrophysiology properties are quite distinct from neonatal and adult cardiac muscle. In particular, the embryonic action potentials are characterized by more depolarized maximum diastolic potentials (MDP) and "slow" type action potentials based on low dV/dtmax (about 5 to about 30 V/sec).

In another embodiment, stem or progenitor cells are seeded onto a perfusion decellularized cardiac ECM scaffold and cultured for a time and under conditions that result in fully functional cardiac cells, e.g., atrial nodal, or ventricular cardiomyocytes. In another embodiment, partially differentiated pancreatic cells derived from ES or iPS cells are seeded onto a perfusion decellularized pancreatic ECM scaffold and cultured for a time and under conditions that result in fully functional alpha cells, PP cells, delta cells, epsilon cells, and/or beta cells, e.g., where alpha cells produce glucagon, beta cells produce insulin and amylin, delta cells produce somatostatin, PP cells produce pancreatic polypeptide, epsilon cells produce ghrelin.

In yet another embodiment, stem or progenitor cells are seeded onto a perfusion decellularized pancreatic ECM scaffold and cultured for a time and under conditions that result in fully functional beta cells. In one embodiment, the culturing results in greater than 35% of the cells expressing insulin in response to glucose stimulation.

In another embodiment, partially differentiated liver cells derived from ES or iPS cells are seeded onto a perfusion decellularized liver ECM scaffold and cultured for a time and under conditions that result in fully functional liver cells, e.g., cells that in the presence of added ammonia, lidocaine, and/or diazepam metabolize reagents, or cells that produce albumin and urea. In another embodiment, stem or progenitor cells are seeded onto a perfusion decellularized liver ECM scaffold and cultured for a time and under conditions that result in fully functional liver cells.

In another embodiment, a population of fully differentiated or primary cells are cultured on a perfusion decellularized ECM scaffold under conditions that maintain the function of the introduced cells.

The ECM organ or tissue matrices may be obtained from any source including, without limitation, heart, liver, lungs, skeletal muscles, brain, pancreas, spleen, kidneys, uterus, eye, spinal cord, whole muscle, or bladder, or any portion thereof (e.g., an aortic valve, a mitral valve, a pulmonary valve, a tricuspid valve, a pulmonary vein, a pulmonary artery, coronary vasculature, septum, a right atrium, a left atrium, a right ventricle, or a left ventricle). A solid organ refers to an organ that has a "substantially closed" vasculature system. A "substantially closed" vasculature system with respect to an organ means that, upon perfusion with a liquid, the majority of the liquid is contained within the solid organ and does not leak out of the solid organ, assuming the major vessels are cannulated, ligated, or otherwise restricted. Despite having a "substantially closed" vasculature system, many of the organs listed above have defined "entrance" and "exit" vessels which are useful for introducing and moving the liquid throughout the organ during perfusion. In addition, other types of vascularized organs or tissues such as, for example, all or portions of joints (e.g., knees, shoulders, or hips), anus, trachea, or spinal cord, can be perfusion decellularized. Further, avascular tisssues such as, for example, cartilage or cornea, may be decellularized when part of a larger vascularized structures such as a whole leg.

Perfusion decellularized matrices of organs with a substantially closed vascular system are particularly useful because perfusion decellularization preserves the intact matrix and microenvironment, including an intact vascular and microvascular system, that vascular system may be utilized to deliver cells as well as nutrients and/or differentiation or maintenance factors, to the cells *in vitro.* Cells and nutrients and/or other factors may be delivered by other means, e.g., injection, or passive means, or a combination thereof. In one embodiment, a cell population of interest is perfused into the perfusion decellularized organ ECM allowing for the seeding into the interstitial space or matrix outside of the vascular conduits. This includes the active migration and/or homing of cells to their native microstructure, e.g. the homing of beta cells to islet structures within the pancreas matrix. In one embodiment, a cell population of interest is perfused into the perfusion decellularized ECM followed by a second cell population, e.g., beta cell population, followed by an endothelial cell population, where the endothelial cells remain in the vascular conduits as in their native microenvironment. In another embodiment, two or more cell populations are combined and perfused together. In another embodiment, two or more distinct cell populations are introduced serially through either perfusion, direct injection or a combination of both. For example, hepatocytes may be introduced to a perfusion decellularized liver matrix either through perfusion or injection followed by the seeding of endothelial cells. In another embodiment, perfusion decellularized liver matrix is seeded with fetal liver cells including endothelial and epithelial cells. In another embodiment, fetal liver cells are seeded in the matrix followed by endothelial cells. In one embodiment, fetal lung cells containing a mixture of all lung cell types including epithelial, endothelial and interstitial lineages, are seeded through perfusion into the lung vasculature and airway of a perfusion decellularized lung, for instance, to create a functional lung construct such as one that provides for further maturation of the isolated lung cells. In one embodiment, a perfusion decellularized heart from a small mammal, e.g., a rat, rabbit, mouse, guinea pig or ferret, is perfused with partially differentiated human cardiomyocytes, human cardiac fibroblasts, human smooth muscle cells, and human endothelial cells to create a beating miniaturized human heart, e.g., for pharmaceutical testing.

The cells may be introduced in media that support the proliferation, metabolism, and/or differentiation of the cells. Alternatively, after the cells have populated the ECM, the medium is changed to one that supports the proliferation, metabolism and differentiation of the cells. The cultured cells may exist in the ECM at physiological cell densities and, in the presence of media that support the proliferation, metabolism, and/or differentiation of the cells and/or the appropriate microenvironment in the ECM, allow for the maintenance and/or functional differentiation of the cells.

In one embodiment, primary cardiac cells are cultured on perfusion decellularized cardiac matrix so as to maintain cardiac-specific expression and function, for instance, as measured by qRT-PCR and patch clamping. When cultured on perfusion decellularized cardiac matrix, contractile function on the introduced primary cardiac cells is maintained for at least 2 weeks. Moreover, those cells are responsive to electrical stimulation and have synchronous contractions. This is in contrast to culturing conditions that do not include ECM scaffolds, where 50 to 70% of primary cardiomyocytes are lost during the first week of culture with a continual decline in their electrophysiology properties (Exp Physiol., 2008 Mar;93(3):370-82. Epub 2007 Dec 21.) and loss of contractile function by as early as day 2.

In one embodiment, partially differentiated cardiac cells, including those derived from ES or iPS cells, are cultured on perfusion decellularized matrix for a time and under conditions that allow for the maturation of cardiac-specific phenotypes. For example, cells are cultured under conditions on perfusion decellularized atrial tissue that yield atrial-specific phenotypes or are cultured under conditions on perfusion decellularized ventricular tissue that yield ventricle-specific phenotypes, as determined by gene expression and/or electrophysiology. In one embodiment, partially differentiated beta cells cultured on a perfusion decellularized pancreatic matrix result in cells capable of insulin regulation and release in respect to various glucose or electrical triggers.

The cells and ECM may be xenogeneic or allogeneic. In one embodiment, partially or completely differentiated human cells and a perfusion decellularized organ or tissue from a small animal, e.g., a nonhuman mammal, can be combined. In one example, a perfusion decellularized liver matrix from a human or rabbit is seeded with partially differentiated human ES derived hepatocyte cells providing allogeneic or xenogeneic, respectively, cell seeded matrices which may be employed for drug or toxicology testing.

Thus, the invention also provides a system for pharmacological testing which employs a recellularized matrix of the invention. Further provided is a system to supply fully differentiated cells for *in vitro* or *in vivo* uses, where the cells are isolated from a recellularized matrix of the invention.

In one embodiment, the invention provides a method to screen for bioactive agents. The method includes contacting one or more agents and a recellularized matrix of the invention; and detecting or determining whether the one or more agents alter metabolism, expression of one or more gene products or the phenotype of the cells in the matrix. In one embodiment, the matrix is from a mammalian heart, pancreas, liver, kidney or lung. In one embodiment, the cells in the matrix are functional cardiac cell types, functional hepatocytes or functional beta-cells. In one embodiment, differentiated functional cardiac cells in the cardiac matrix have atrial-specific action potentials or ventricle-specific action potentials. In one embodiment, differentiated functional hepatocytes in the liver matrix express albumin, express HepPar1, and/or deposit glycogen, or release albumin and urea, or any combination thereof. In one embodiment, differentiated beta cells in the matrix release insulin in response to glucose stimulation. In one embodiment, the cells and the perfusion decellularized organ or tissue are allogeneic. In one embodiment, the cells and the perfusion decellularized matrix are xenogeneic. In one embodiment, the cells in the matrix include human hepatocytes and the perfusion decellularized matrix is from a nonhuman mammal.

In one embodiment, the invention provides a method to provide mature cells *in vitro.* The method includes providing a recellularized matrix of the invention; allowing for differentiation or maturation of the cells in the matrix; and isolating the differentiated or mature cells from the matrix. In one embodiment, the matrix is from a mammalian heart, pancreas, liver, kidney or lung. In one embodiment, the cells in the matrix are functional cardiac cell types, functional hepatocytes or functional beta-cells. In one embodiment, differentiated functional cardiac cells in the cardiac matrix have atrial-specific action potentials or ventricle-specific action potentials. In one embodiment, differentiated functional hepatocytes in the liver matrix express albumin, express HepPar1, and/or deposit glycogen, or release albumin and urea, or any combination thereof. In one embodiment, differentiated beta cells in the matrix release insulin in response to glucose stimulation. In one embodiment, the cells and the perfusion decellularized organ or tissue are allogeneic. In one embodiment, the cells and the perfusion decellularized matrix are xenogeneic. In one embodiment, the cells in the matrix include human hepatocytes and the perfusion decellularized matrix is from a nonhuman mammal.

### Brief Description of the Figures

Figure 1A shows a photograph of a porcine liver that was perfusion decellularized.
Figures 1B-C show a scanning electron microscope (SEM) photograph of a vessel and the parenchymal matrix, respectively, of the perfusion decellularized porcine liver.
Figure 2 provides a gross view of an immersion decellularized rat liver, in which fraying of the matrix can be seen at both low (left) and high (right) magnification.
Figure 3 shows SEM photographs of immersion decellularized rat liver (A and B) and perfusion decellularized rat liver (C and D).
Figure 4 provides histology of immersion decellularized liver (A, H&E staining; B, Trichrome staining) and perfusion decellularized liver (C, H&E staining; D, Trichrome staining).
Figure 5 illustrates a comparison between immersion decellularization (top row) and perfusion decellularization (bottom row) of a rat heart.
Figure 6 shows a comparisons between immersion decellularization (top row) versus perfusion decellularization (bottom row) using rat kidney.
Figure 7 shows SEM photographs of decellularized kidney
Figure 8A shows a SEM photograph of a perfusiondecellularized heart, while Figure 8B shows a SEM photograph of an immersion decellularized heart.

### Detailed Description of the Invention

The present invention provides for engineering of cells and ECMs that, through physical as well as molecular interactions, direct control of cell behavior by controlling the environment of those cells. In particular, the present invention provides engineered organs, tissues or bioreactors having perfusion decellularized ECM implanted with a population of cells, including combinations of cells, and subjected to culture conditions, e.g., including perfusion of soluble mediators, which ECM structure and culture conditions result in functional cells. In particular, the invention provides for improved regulation of cell differentiation, growth, and phenotypic expression of stem cells, both adult and embryonic, and partially differentiated progenitor cells, and improved maintenance of differentiated cell types. It also includes the growth and functional maintenance of primary cells including fetal derived cells, e.g., organ-specific cells obtained from fetal cells or neonate cells (for instance, cells that are committed to a specific lineage but are not terminally differentiated). The invention overcomes limited cellular differention that yields a low percentage of a specific phenotype and/or obtains only a neonatal or early developmental phenotype. For example, currently cardiac differentiation of iPS cells (Zhang et al., Circ Res., 27;104(4):e30-41.(2009) Epub 2009 Feb 12) yields only 13% atrial like cardiomyocytes. In contrast, the use of the systems of the invention result in an increased percentage of differentiated cells, e.g., to greater than 30% when iPS cells were cultured in or on atrial-specific ECM. It is also possible to obtain a more mature or adult like phenotype using the systems of the invention. For cardiac differentiation, this is characterized by various electrophysiology properties where embryonic action potentials are characterized by more depolarized maximum diastolic potentials (MDP) and "slow" type action potentials based on low dV/dtmax (about 5 to about 30 V/sec). In contrast to the use of *in vivo* transplanation to differentiation for hES cells into insulin producing cells, the present invention provides for the use of pancreas ECM to achieve differentiation *in vitro* to functional beta cells capable of insulin regulation in response to glucose stimulation. In one embodiment, the differentiated cells of the invention have at least 20% of the function of corresponding normal cells *in vivo.*

In particular, the perfusion decellularized ECMs are suitable for 3D culture of primary and stem cells *in vitro,* and for organ or tissue formation, as the ECM provides appropriate biological cues needed to recapitulate the complexity of a given ECM environment and the ability to be continuously perfused. The resulting bioreactor may be employed, for instance, to provide for stem or progenitor cell expansion with control of differentiation, and xenografts that can be readily recovered from the cellularized constructs after expansion, differentiation, and/or matrix remodeling have occurred, or organs, e.g., vascularized structures of organs, or tissues that contain functional cell populations that can be used for toxicology testing.

### Perfusion Decellularized ECM

Studies have shown that connective tissue cells behave very differently in 3D as opposed to 2D cultures (Cukierman et al., Science, 294:1708 (2001)). For example, culture of fibroblasts on flat substrates induces a polarity that does not occur *in vivo.* Further, when fibroblasts and other cell types are cultured in 3D tissue-derived matrices, they develop mature integrin-containing focal adhesion complexes within minutes that resemble the complexes found *in vivo,* whereas only primitive adhesion complexes develop in 2D cultures or even simple 3D type I collagen gels or Matrigel. These adhesion complexes are required for appropriate growth factor-activated receptor signaling and rapid (within 5 minutes) initiation of synthesis of their own ECM components and factors that alter the ECM (Cukierman et al., 2001; Abbott, Nature, 424:870 (2003)). In addition, cells in ECM culture deposit autocrine growth factors into tissue-derived matrices, a process that may be required for appropriate presentation of the growth factor to target cells. Such factors are mainly secreted into the culture medium in 2D cultures.

As mentioned above, physical interactions with the ECM, in addition to chemical, molecular (e.g., soluble mediators), or genetic (cell-type) factors, may regulate cell fate. For example, ECM-based control of the cell may occur through multiple physical mechanisms, such as ECM geometry at the micro- and nanoscale, ECM elasticity, or mechanical signals transmitted from the ECM to the cells.

The invention includes the use of engineered perfusion decellularized ECMs that allow for better control of cell behavior, e.g., from adult or embryonic stem cells, through physical as well as molecular interactions relative to systems that employ purified components, such as purified collagens and adhesive proteins such as fibronectin. The perfusion decellularized matrices of the invention mimic the intricate and highly ordered nature of native ECM and the likely reciprocal interaction between cells and the ECM. In particular, the ECM may provide tissue-specific cues to stem or progenitor cells. In particular, distinct matrix proteins may be important for the specificity of ECM via their contribution to the architecture of the ECM or via their ability to interact with growth factors and/or the resident cells themselves.

Perfusion decellularization of tissue or organ ECM provides an intact ECM that has the ability to provide the structural, biochemical, and mechanical properties to enable functional cell differentiation and maintenance. Thus, perfusion decellularization of organs allows organs to serve as a tissue/organ specific bioreactor for stem or progenitor cell differentiation. Moreover, perfusion decellularization of organ or tissue ECM is superior to immersion in terms of preserving an intact matrix with structural and biochemical cues, including intact vasculature. In addition, perfusion decellularization provides advantages relative to immersion decellularization when tissue or organ thickness exceeds about 2 mm in thickness.

### Decellularization of Organs or Tissues

Decellularization generally includes the following steps: stabilization of the solid organ, e.g., a vascuarlized structure thereof, or tissue, decellularization of the solid organ or tissue, renaturation and/or neutralization of the solid organ or tissue, washing the solid organ, degradation of any DNA remaining on the organ, disinfection of the organ or tissue and homeostasis of the organ.

The initial step in decellularizing an organ vascularized structure or tissue is to cannulate the organ or tissue. The vessels, ducts, and/or cavities of an organ or tissue tissue may be cannulated using methods and materials known in the art. Next, the cannulated organ vascuarlized structure or tissue is perfused with a cellular disruption medium. Perfusion through an organ can be multidirectional (e.g., antegrade and retrograde).

Langendorff perfusion of a heart is routine in the art, as is physiological perfusion (also known as four chamber working mode perfusion). See, for example, Dehnert, The Isolated Perfused Warm-Blooded Heart According to Langendorff, In Methods in Experimental Physiology and Pharmacology: Biological Measurement Techniques V. Biomesstechnik-Verlag March GmbH, West Germany, 1988.

Briefly, for Langendorff perfusion, the aorta is cannulated and attached to a reservoir containing physiological solution to allow the heart to function outside of the body for a specified duration of time. To achieve perfusion decellularization the protocol has been modified to perfuse a cellular disruption medium delivered in a retrograde direction down the aorta either at a constant flow rate delivered, for example, by an infusion or roller pump or by a constant hydrostatic pressure pump. In both instances, the aortic valves are forced shut and the perfusion fluid is directed into the coronary ostia (thereby perfusing, via antegrade, the entire ventricular mass of the heart), which then drains into the right atrium via the coronary sinus. For working mode perfusion, a second cannula is connected to the left atrium and perfusion can be changed to retrograde.

In one embodiment, a physiological solution includes phosphate buffer saline (PBS). In one embodiment, the physiological solution is a physiologically compatible buffer supplemented with, e.g., nutritional supplements (for instance, glucose). For example, for heart, the physiological solution may be Modified Krebs-Henseleit buffer having 118 mM NaCl, 4.7 mM KCl, 1.2 mM MgSO₄, 1.2 mM KH₂PO₄, 25 mM NaHCO₃, 11 mM glucose, 1.75 mM CaCl₂, 2.0 mM pyruvate and 5 U/L insulin; or Krebs buffer containing 118 mM NaCl, 4.7 mM KCl, 25 mM NaHCO₃, 1.2 mM MgSO₄, 1.2 mM KH₂PO₄, 2 mM CaCl₂ gassed with 95% O₂, 5% CO₂. Hearts may be perfused with glucose (e.g., about 11 mM) as a sole substrate or in combination with about 1 or 1.2 mM palmitate. For kidney, the physiological solution may be KPS-1® Kidney Perfusion Solution. For liver, the physiological solution may be
Krebs-Henseleit buffer having 118 mM NaCl, 4.7 mM KCI, 1.2 mM MgSO₄, 1.2 mM KH₂PO₄, 26 mM NaHCO₃, 8 mM glucose, and 1.25 mM CaCl₂ supplemented with 2% BSA.

Methods are known in the art for perfusing other organ or tissues. By way of example, the following references describe the perfusion of lung, liver, kidney, brain, and limbs. Van Putte et al., Ann. Thorac. Surg., 74(3):893 (2002); den Butter et al., Transpl. Int., 8:466 (1995); Firth et al., Clin. Sci. (Lond.), 77(6):657 (1989); Mazzetti et al., Brain Res., 999(1):81 (2004); Wagner et al., J. Artif. Origans, 6(3):183 (2003).

One or more cellular disruption media may be used to decellularize an organ or tissue. A cellular disruption medium generally includes at least one detergent such as but not limited to SDS, PEG, CHAPS or Triton X. A cellular disruption medium can include water such that the medium is osmotically incompatible with the cells. Alternatively, a cellular disruption medium can include a buffer (e.g., PBS) for osmotic compatibility with the cells. Cellular disruption media also may include enzymes such as, without limitation, one or more collagenases, one or more dispases, one or more DNases, or a protease such as trypsin. In some instances, cellular disruption media also or alternatively may include inhibitors of one or more enzymes (e.g., protease inhibitors, nuclease inhibitors, and/or collegenase inhibitors).

In certain embodiments, a cannulated organ or tissue may be perfused sequentially with two different cellular disruption media. For example, the first cellular disruption medium may include an anionic detergent such as SDS and the second cellular disruption medium can include an ionic detergent such as Triton X. Following perfusion with at least one cellular disruption medium, a cannulated organ or tissue may be perfused, for example, with wash solutions and/or solutions containing one or more enzymes such as those disclosed herein.

Alternating the direction of perfusion (e.g., antegrade and retrograde) may assist in decellularizing the entire organ or tissue. Decellularization generally decellularizes the organ from the inside out, resulting in very little damage to the ECM. An organ or tissue may be decellularized at a suitable temperature between 4 and 40°C. Depending upon the size and weight of an organ or tissue and the particular detergent(s) and concentration of detergent(s) in the cellular disruption medium, an organ or tissue generally is perfused from about 0.05 hours to about 5 hours, per gram of solid organ or tissue (generally > 50 grams), or about 2 hours to about 12 hours, per gram of solid organ or tissue for organs (generally < 50 grams), with cellular disruption medium. Including washes, an organ may be perfused for up to about 0.75 hours to about 10 hours per gram of solid organ or tissue (generally >50 grams), or about 12 hours to about 72 hours, per gram of tissue (generally <50 grams). Decellularization time is dependent upon the vascular and cellular density of the organ or tissue with limited scaling for overall mass. Therefore, as general guidance the time ranges and masses above are provided. Perfusion generally is adjusted to physiologic conditions including pulsatile flow, rate and pressure.

A decellularized organ or tissue has the extracellular matrix (ECM) component of all or most regions of the organ or tissue, including ECM components of the vascular tree. ECM components can include any or all of the following: fibronectin, fibrillin, laminin, elastin, members of the collagen family (e.g., collagen I, III, and IV), ECM associated growth proteins including growth factors and cytokines, glycosaminoglycans, ground substance, reticular fibers and thrombospondin, which can remain organized as defined structures such as the basal lamina. Successful decellularization is defined as the absence of detectable myofilaments, endothelial cells, smooth muscle cells, and nuclei in histologic sections using standard histological staining procedures or removal of over 97% of detectable DNA as measured by fluorometric assay. Residual cell debris may be removed from the decellularized organ or tissue.

The morphology and the architecture of the ECM is maintained during and following the process of decellularization. "Morphology" as used herein refers to the overall shape of the organ, tissue or of the ECM, while "architecture" as used herein refers to the exterior surface, the interior surface, and the ECM therebetween.

The morphology and architecture of the ECM may be examined visually and/or histologically. For example, the basal lamina on the exterior surface of a solid organ or within the vasculature of an organ or tissue should not be removed or significantly damaged due to perfusion decellularization. In addition, the fibrils of the ECM should be similar to or significantly unchanged from that of an organ or tissue that has not been decellularized.

One or more compounds may be applied in or on a decellularized organ or tissue to, for example, preserve the decellularized organ, or to prepare the decellularized organ or tissue for recellularization and/or to assist or stimulate cells during the recellularization process. Such compounds include, but are not limited to, one or more growth factors (e.g., VEGF, DKK-1, FGF, BMP-1, BMP-4, SDF-1, IGF, and HGF), immune modulating agents (e.g., cytokines, glucocorticoids, IL2R antagonist, leucotriene antagonists), and/or factors that modify the coagulation cascade (e.g., aspirin, heparin-binding proteins, and heparin). In addition, a decellularized organ or tissue may be further treated with, for example, irradiation (e.g., UV, gamma) to reduce or eliminate the presence of any type of microorganism remaining on or in a decellularized organ or tissue.

### Exemplary Perfusion Decellularization of Heart

### PEG Decellularization Protocol

Hearts are washed in 200 ml PBS containing 100 U/mL penicillin, 0.1 mg/mL Streptomycin, 0.25 µg/mL Amphotericin B, 1000 U of hepatin, and 2 mg of Adenocard with no recirculation. Hearts are then decellularized with 35 ml polyethyleneglycol (PEG; 1 g/mL) for up to 30 minutes with manual recirculation. The organ is then washed with 500 mL PBS for up to 24 hours using a pump for recirculation. The washing step is repeated at least twice for at least 24 hours each time. Hearts are exposed to 35 ml DNase I (70 U/mL) for at least 1 hour with manual recirculation. The organs are washed again with 500 ml PBS for at least 24 hours.

### Triton X and Trypsin Decellularization Protocol

Hearts are washed in 200 ml PBS containing 100 U/mL Penicillin, 0.1 mg/mL Streptomycin, 0.25 µg/mL Amphotericin B, 1000 U of hepatin, and 2 mg of Adenocard for at least about 20 minutes with no recirculation. Hearts are then decellularized with 0.05% Trypsin for 30 minutes followed by perfusion with 500 mL PBS containing 5% Triton-X and 0.1% ammonium-hydroxide for about 6 hours. Hearts are perfused with deionized water for about 1 hour, and then perfused with PBS for 12 hours. Hearts are then washed 3 times for 24 hours each time in 500 mL PBS using a pump for recirculation. The hearts are perfused with 35 ml DNase I (70 U/mL) for 1 hour with manual recirculation and washed twice in 500 mL PBS for at least about 24 hours each time using a pump for recirculation.

### 1% SDS Decellularization Protocol

Hearts are washed in 200 mL PBS containing 100 U/mL Penicillin, 0.1 mg/mL Streptomycin, 0.25 µg/mL Amphotericin B, 1000 U of hepatin, and 2 mg of Adenocard for at least about 20 minutes with no recirculation. The hearts are decellularized with 500 mL water containing 1% SDS for at least about 6 hours using a pump for recirculation. The hearts are then washed with deionized water for about 1 hour and washed with PBS for about 12 hours. The hearts are washed three times with 500 mL PBS for at least about 24 hours each time using a pump for recirculation. The heart is then perfused with 35 ml DNase I (70 U/mL) for about 1 hour using manual recirculation, and washed three times with 500 mL PBS for at least about 24 hours each time using a pump for recirculation.

### Triton X Decellularization Protocol

Hearts are washed with 200 mL PBS containing 100 U/mL Penicillin, 0.1 mg/ml Streptomycin, 0.25 µg/mL Amphotericin B, 1000 U of hepatin, and 2 mg of Adenocard (adenosine) for at least about 20 minutes with no recirculation. Hearts are then decellularized with 500 mL water containing 5% Triton X and 0.1% ammonium hydroxide for at least 6 hours using a pump for recirculation. Hearts are then perfused with deionized water for about 1 hour and then with PBS for about 12 hours. Hearts are washed by perfusing with 500 mL PBS 3 times for at least 24 hours each time using a pump for recirculation. Hearts are then perfused with 35 ml DNase I (70 U/mL) for about 1 hour using manual recirculation, and washed three times in 500 ml PBS for about 24 hours each time.

Hearts may be perfused at a coronary perfusion pressure of 60 cm H₂O. Although not required, the hearts may be mounted in a decellularization chamber and completely submerged and perfused with PBS containing antibiotics for 72 hours in recirculation mode at a continuous flow of 5 mL/minute to wash out as many cellular components and detergent as possible.

### Detection of Cardiac Decellularization

Successful decellularization may be measured by the lack of myofilaments and nuclei in histologic sections. Successful preservation of vascular structures may be assessed by perfusion with 2% Evans Blue prior to embedding tissue sections. Highly efficient decellularization is observed when a heart is first perfused antegradely with an ionic detergent (1% sodium-dodecylsulfate (SDS), approximately 0.03 M) dissolved in deionized H₂O at a constant coronary perfusion pressure and then perfused antegradely with a non-ionic detergent (1% Triton X-100) to remove the SDS and presumably to renature the extracellular matrix (ECM) proteins. Intermittently, the heart may be perfused retrogradely with phosphate buffered solution to clear obstructed capillaries and small vessels.

To demonstrate intact vascular structures following decellularization, a decellularized heart may be stained via Langendorff perfusion with Evans Blue to stain vascular basement membrane and quantify macro- and micro-vascular density. Further, polystyrene particles may be perfused into and through a heart to quantify coronary volume, the level of vessel leakage, and to assess the distribution of perfusion by analyzing coronary effluent and tissue sections. A combination of three criteria are assessed and compared to isolated non-decellularized heart: 1) an even distribution of polystyrene particles, 2) significant change in leakiness at some level 3) microvascular density.

Fiber orientation may be assessed by the polarized-light microscopy technique of Tower et al. (Ann Biomed Eng., 30(10):1221 (2002), which can be applied in real-time to a sample subjected to uniaxial or biaxial stress. During Langendorff perfusion, basic mechanical properties of the decellularised ECM are recorded (compliance, elasticity, burst pressure) and compared to freshly isolated hearts.

### Exemplary Perfusion Decellularization of Liver

For liver isolation, the caval vein is exposed through a median laparotomy, dissected and canulated using a mouse aortic canula (Radnoti Glass, Monrovia, Calif.). The hepatic artery and vein and the bile duct are transsected and the liver was carefully removed from the abdomen and submerged in sterile PBS (Hyclone, Logan, Utah) to minimize pulling force on portal vein. 15 minutes of heparinized PBS perfusion is followed by 2-12 hours of perfusion with 1% SDS (Invitrogen, Carlsbad, Calif.) in deionized water and 15 minutes of 1% Triton-X (Sigma, St. Louis, Mo.) in deionized water. The liver is then continuously perfused with antibiotic containing PBS (100 U/ml penicillin-G (Gibco, Carlsbad, Calif.), 100 U/ml streptomycin (Gibco, Carlsbad, Calif.), 0.25 µg/ml Amphotericin B (Sigma, St. Louis, Mo.)) for 124 hours.

120 minutes of SDS perfusion followed by perfusion with Triton-X 100 are sufficient to generate a completely decellularized liver. Movat pentachrome staining of decellularized liver confirms retention of characteristic hepatic organization with central vein and portal space containing hepatic artery, bile duct and portal vein.

### Recellularization of Organs or Tissues

A decellularized organ or tissue is contacted with a population of cells, either differentiated (mature or primary) cells, stem cells, or partially differentiated cells. Thus, the cells can be totipotent cells, pluripotent cells, or multipotent cells, and can be uncommitted or committed, and may be single-lineage cells. The cells may be undifferentiated cells, partially differentiated cells, or fully differentiated cells including fetal derived cells. Cells may include progenitor cells, precursor cells, or "adult" derived stem cells including umbilical cord cells and fetal stem cells. Cells useful in the matrices of the invention include embryonic stem cells (as defined by the National Institute of Health (NIH); see, for example, the Glossary at stemcells.nih.gov on the World Wide Web) and iPS cells.

Examples of cells that can be used to recellularize an organ or tissue include, without limitation, embryonic stem cells, umbilical cord blood cells, tissue-derived stem or progenitor cells, bone marrow-derived step or progenitor cells, blood-derived stem or progenitor cells, mesenchymal stem cells (MSC), skeletal muscle-derived cells, multipotent adult progentitor cells (MAPC), or iPS cells Additional cells that can be used include cardiac stem cells (CSC), multipotent adult cardiac-derived stem cells, cardiac fibroblasts, cardiac microvasculature endothelial cells, aortic endothelial cells, coronary endothelial cells, microvascular endothelial cells, venous endothelial cells, arterial endothelial cells, smooth muscle cells, cardiomyocytes, hepatocytes, beta-cells, keratinocytes, purkinji fibers, neurons, bile duct epithelial call, islet cells, pneumocytes, clara cells, brush boarder cells, or podocytes. Bone marrow-derived stem cells such as bone marrow mononuclear cells (BM-MNC), endothelial or vascular stem or progenitor cells, and peripheral blood-derived stem cells such as endothelial progenitor cells (EPC) may also be used as cells.

The number of cells that are introduced into and onto a perfusion decellularized scaffold may depend both the organ (e.g., which organ, the size and weight of the organ) or tissue and the type and developmental stage of the regenerative cells. Different types of cells may have different tendencies as to the population density those cells will reach. Similarly, different organ or tissues may be cellularized at different densities. By way of example, a decellularized organ or tissue can be "seeded" with at least about 1,000 (e.g., at least 10,000, 100,000, 1,000,000, 10,000,000, or 100,000,000) cells; or can have from about 1,000 cells/mg tissue (wet weight, e.g., prior to decellularization) to about 10,000,000 cells/mg tissue (wet weight) attached thereto.

Cells can be introduced ("seeded") into a decellularized organ or tissue by injection into one or more locations. In addition, more than one type of cell may be introduced into a decellularized organ or tissue. For example, a population of differentiated cell types can be injected at multiple positions in a decellularized organ or tissue or different cell types may be injected into different portions of a decellularized organ or tissue. Alternatively, or in addition to injection, cells or a cocktail of cells may be introduced by perfusion into a cannulated decellularized organ or tissue. For example, cells can be perfused into a decellularized organ using a perfusion medium, which can then be changed to an expansion and/or differentiation medium to induce growth and/or differentiation of the cells. Location specific differentiation may be achieved by placing cells into the various locations within the organ, e.g., into regions of the heart, such as, atrial, ventricular or nodal.

During recellularization, an organ or tissue is maintained under conditions in which at least some of the cells can multiply and/or differentiate within and on the decellularized organ or tissue. Those conditions include, without limitation, the appropriate temperature and/or pressure, electrical and/or mechanical activity, force, the appropriate amounts of O₂ and/or CO₂, an appropriate amount of humidity, and sterile or near-sterile conditions. During recellularization, the decellularized organ or tissue and the regenerative cells attached thereto are maintained in a suitable environment. For example, the cells may require a nutritional supplement (e.g., nutrients and/or a carbon source such as glucose), exogenous hormones or growth factors, and/or a particular pH.

Cells may be allogeneic to a decellularized organ or tissue (e.g., a human decellularized organ or tissue seeded with human cells), or cells may be xenogeneic to a decellularized organ or tissue (e.g., a pig decellularized organ or tissue seeded with human cells). "Allogeneic" as used herein refers to cells obtained from the same species as that from which the organ or tissue originated (e.g., related or unrelated individuals), while "xenogeneic" as used herein refers to cells obtained from a species different than that from which the organ or tissue originated.

Stem or progenitor media may contain a variety of components including, for example, KODMEM medium (Knockout Dulbecco's Modified Eagle's Medium), DMEM, Ham's F12 medium, FBS (fetal bovine serum), FGF2 (fibroblast growth factor 2), KSR or hLIF (human leukemia inhibitory factor). The cell differentiation media may also contain supplements such as L-Glutamine, NEAA (non-essential amino acids), P/S (penicillin/streptomycin), N2, B27 and beta-mercaptoethanol. It is contemplated that additional factors may be added to the cell differentiationmedia, including, but not limited to, fibronectin, laminin, heparin, heparin sulfate, retinoic acid, members of the epidermal growth factor family (EGFs), members of the fibroblast growth factor family (FGFs) including FGF2, FGF7, FGF8, and/or FGF10, members of the platelet derived growth factor family (PDGFs), transforming growth factor (TGF)/bone morphogenetic protein (BMP)/growth and differentiation factor (GDF) factor family antagonists including but not limited to noggin, follistatin, chordin, gremlin, cerberus/DAN family proteins, ventropin, high dose activin, and amnionless or variants or functional fragments thereof. TGF/BMP/GDF antagonists could also be added in the form of TGF/BMP/GDF receptor-Fc chimeras. Other factors that may be added include molecules that can activate or inactivate signaling through Notch receptor family, including but not limited to proteins of the Delta-like and Jagged families as well as inhibitors of Notch processing or cleavage, or variants or functional fragments thereof. Other growth factors may include members of the insulin like growth factor family (IGF), insulin, the wingless related (WNT) factor family, and the hedgehog factor family or variants or functional fragments thereof. Additional factors may be added to promote mesendoderm stem/progenitor, endoderm stem/progenitor, mesoderm stem/progenitor, or definitive endoderm stem/progenitor proliferation and survival as well as survival and differentiation of derivatives of these progenitors.

In one embodiment, perfusion decellularized matrices are combined with iPS or ES cells differentiated using the embryoid body (EB) method. For example, human iPS cell lines reprogrammed by transduction, e.g., lentiviral-mediated transduction, of transcription factors (*OCT4, SOX2, NANOG* and *LIN28;* Oct3/4, Sox2, Klf4, and c-Myc; or Oct3/4, Sox2, and Klf4) are employed. iPS clones of fetal origin or of newborn origin may be employed. Human ES cell lines may also be employed. iPS cells and ES cells may be maintained on irradiated mouse embryonic fibroblasts (MEFs) at a density of 19,500 cells/cm² in 6-well culture plates (Nunc) in DMEM/F12 culture medium supplemented with 20% KnockOut serum replacer (Invitrogen), 0.1 mmol/L nonessential amino acids, 1 mmol/L L-glutamine, and 0.1 mmol/L β-mercaptoethanol (Sigma). In addition, the medium may be supplemented with 100 ng/mL, zebrafish basic fibroblast growth factor for iPS cells, and with 4 ng/mL human recombinant basic fibroblast growth factor (Invitrogen) for hES cells. iPS and ES cell lines may also be maintained on gelatinized 100-mm dishes in DMEM (Sigma-Aldrich) containing 15% fetal calf serum (FCS; Sigma-Aldrich), 0.1 µmol/L 2-mercaptoethanol (2ME), and 1,000 units/ml LIF (Chemicon International). For differentiation, these cells may treated with 0.25% Trypsin/ethylenediaminetetraacetic acid (GIBCO), and transferred to gelatinized 6-well plates in α-minimum essential medium (GIBCO) supplemented with 10% FCS and 0.05 µmol/L 2ME, at a concentration of 3 × 10⁴ cells/well.

Colonies may be detached from culture plates by incubating with 1 mg/mL dispase (Gibco) solution at 37°C for 8 to 15 minutes and placed in ultralow attachment plates in suspension culture, e.g., for 4 days. During suspension culture, the medium may be changed at day 1 followed by culture for another 3 days without medium change. EBs are then plated on 0.1% gelatin-coated culture plates, e.g., at the density or 50 to 100 EBs per well, or in the perfusion decellularized ECM and cultured in differentiation medium (e.g., changed daily).

In some instances, an organ or tissue generated by the methods described herein is to be transplanted into a patient. In those cases, the cells used to recellularize a decellularized organ or tissue can be obtained from the patient such that the cells are "autologous" to the patient. Cells from a patient can be obtained from, for example, blood, bone marrow, tissues, or organs at different stages of life (e.g., prenatally, neonatally or perinatally, during adolescence, or as an adult) using methods known in the art. Alternatively, cells used to recellularize a decellularized organ or tissue may be syngeneic (i.e., from an identical twin) to the patient, cells can be human lymphocyte antigen (HLA)-matched cells from, for example, a relative of the patient or an HLA-matched individual unrelated to the patient, or cells can be allogeneic to the patient from, for example, a non-HLA-matched donor.

Irrespective of the source of the cells (e.g., autologous or not), the decellularized solid organ can be autologous, allogeneic or xenogeneic to a patient.

The progress of cells can be monitored during recellularization. For example, the number of cells on or in an organ or tissue can be evaluated by taking a biopsy at one or more time points during recellularization. In addition, the amount of differentiation that cells have undergone can be monitored by determining whether or not various markers are present in a cell or a population of cells. Markers associated with different cells types and different stages of differentiation for those cell types are known in the art, and can be readily detected using antibodies and standard immunoassays. See, for example, Current Protocols in Immunology, 2005, Coligan et al., Eds., John Wiley & Sons, Chapters 3 and 11. Nucleic acid assays as well as morphological and/or histological evaluation can be used to monitor recellularization.

### Exemplary Cardic Cell Differentiation

Differentiation medium for cardiac cells (EB20) may include 80% DMEM/FI2, 0.1 mmol/L nonessential amino acids, 1 mmol/L L-glutamine, 0.1 mmol/L β-mercaptoethanol, and 20% FBS (Gibco. catalog no. 16000-044, lot no. 291526). After 10 days of differentiation, the FBS concentration may be reduced, e.g., to 2%. The number of contracting cells, e.g., EBs, and contraction rates may be measured at, for example, day 10, 20, 30, and 60, e.g., from EB formation, using a microscope with a heated stage (37°), and expression of any of the set of the following markers may be measured: *OCT4, NANOG, NKX2-5, TNNT2, MYH6, ACTN2, MYL7, MYL2, HPPA, PLN, ACTB, GAPDH3, OCT4* (total), *OCT4* (endogenous), *NANOG* (total), and/or *NANOG* (endogenous). Other measures of cardiac lineage differentiation include electrophysiology studies, e.g., differentiation into nodal-, atrial-, and ventricular-like phenotypes may be detected based on action potential characteristics. Both iPS and ES cell-derived cardiomyocytes may also exhibit responsiveness to β-adrenergic stimulation, such as by an increase in spontaneous rate and a decrease in action potential duration.

### Exemplary Pancreatic Cell Linkage Differentiation

Upon activation of *Pdx1* and *Ptf1a,* the pancreatic fate is induced from endoderm progenitors. Pancreatic progenitors give rise to ductal, acini, and endocrine progenitors (the latter give rise to ∈ (ghrelin), PP (pancreatic peptide), β, α (glucagon), and δ (somatastatin) secreting cells). Endocrine progenitors are then differentiated (Ngn3, Hnf6 and Hnf1) into different hormone-secreting cells, α, β, δ, PP, and ∈. Key transcription factors involved in different steps of beta-cell formation are Pax4, Arx, Nkx2.2, Nkx6.1, Pdx1 and Mafa.

Human ES are capable of partial differentiation, e.g., into early or fetal pancreatic endoderm or pancreatic epithelium lineages, as characterized by coexpression of PDX1, FOXA2, HNF6 and NKX6-1 by, e.g., day 12, of *in vitro* differentiation.

To obtain functional beta-cells, perfusable pancreatic ECM scaffold is employed to support beta-cell differentiation in a defined 3D culture environment. The perfused decellularized matrix allows for perfusion through intact vascular network whereas other decelllularization technologies disrupt the vascular network and extracellular matrix thus not allowing for perfusion.

The beta-cell differentiation protocol may be as described in D'Amour et al, Nat. Biotech., 24:1392 (2006), but with modifications at two of the stages. The first modification eliminates cyclopamine (inhibitor of Hedgehog) and substitutes KGF for FGF10 during stage 2 (days 4-6). The other modification, in stage 3, substitutes Noggin for FGF10. The entire differentiation protocol is as follows. Initiated on days 4-6 after passage (depending on culture density), sequential, daily media changes are made for the entire differentiation protocol. After a brief wash in PBS (with Mg/Ca), the cells are cultured in RPMI (without FBS), activin A (100 ng/ml) and Wnt3a (25 ng/ml) for the first day. The next day the medium is changed to RPMI with 0.2% vol/vol FBS and activin A (100 ng/mL), and the cells are cultured for 2 additional days. Next, the cells are briefly washed with PBS (with Mg/Ca) and then cultured in RPMI with 2% vol/vol FBS and KGF (25-50 ng/mL) for 3 days. The medium is changed to DMEM with 1% vol/vol B27 supplement, KAAD-cyclopamine (0.25 M), all-trans retinoic acid (RA, 2 M) and Noggin (50 ng/mL) for 3 days. The medium is changed to DMEM with 1% vol/vol B27 supplement for 3 days.

Alternatively, reference human ES cell lines H1 and H9 and mouse embryonic fibroblasts are described. Human ES cell culture medium includes DMEM/F12 (Invitrogen) with 20% Knockout Serum Replacement (KSR) (Invitrogen) containing 8 ng/mL of bFGF (Invitrogen), Nonessential amino acids (1:100, Invitrogen), 4 mM 1-Glutamine, 0.1 mM 2-Mercaptoethanol (Invitrogen), Penicillin/Streptomycin (1:100, Invitrogen); store at 4°C.

Human ES cell differentiation medium includes (a) Chemical Defined Medium (CDM): 50% IMDM (Invitrogen) plus 50% F12 Nutrient Mixture (Invitrogen), supplemented with Insulin-Transferrin-Selenium-A (1:100, Invitrogen), 450 mM Monothioglycerol (Sigma), and 5 mg/mL Albumin Fraction V (Sigma). (b) Islet Maturation Medium (IMM):DMEM/F12, Insulin-Transferrin-Selenium-A fraction V (Sigma). Factors for human ES cell to insulin-producing cell differentiation are: Activin A (R&D System) 50-100 ng/mL, all-trans Retinoic Acid (Sigma) 10⁻⁶M, bFGF (Invitrogen) 10 ng/mL, and Nicotinamide (Sigma) 10 mM.

hES cell lines are maintained following a typical protocol. Before induction, hES cells are split, e.g., as 1;3 (60% confluent), and replated onto tissue culture dishes, e.g., 1% Matrigel-coated tissue culture dishes, or introduced to ECM. hES cells are incubated with hES culture medium overnight for attachment.

Undifferentiated human ES cells are first cultured in CDM containing Activin A for 4 days. Then, the differentiated cells were further induced with RA in CDM for 4 days and transferred from CDM culture medium into DMEM/F12 islet maturation medium with bFGF added as a pancreatic cell maturation factor for 3 days. Finally, the differentiated cells are switched to DMEM/F12 islet maturation medium containing bFGF and nicotinamide for another 5 days. To induce human ES cells to differentiate into definitive endoderm cells, the medium is changed into CDM or DMEM/F12 medium with 50-100 ng/mL Activin A. After a 4-day differentiation, human ES cells are further induced with 10⁻⁶M RA in CDM or DMEM/F12 medium for another 4 days for pancreatic lineage-specific cells. For insulin producing cells, the pancreatic lineage-specific cells are treated with Activin A and RA and then transferred from CDM or DMEM/F12 medium to IMM containing 10 ng/mL bFGF as a pancreatic cell maturation factor for 3 days. The differentiated cells are switched to IMM containing 10 mM Nicotinamide and 10 ng/mL bFGF for another 3-7 days for insulin-producing cell maturation.

### Exemplary Liver Cell Lineage Differentiation

Hepatocyte differentiation from various stem cell populations is either a multi-step procedure consisting of separate treatments with BMPs and FGFs to commit cells to the hepatic lineage followed by a maturation step which uses dexamethasone and IL6, or a single differentiation step using HGF and EGF. Both of these methods yield hepatocytes-like cells capable of expressing key hepatocyte markers including CXCR4, SOX17, FOXA2, albumin, phosphoenolpyrucate carboxykinase (PCK), glumatime synthetase (GS), and various P450 enzymes. To obtain functional hepatocytes, perfusable liver ECM scaffold is employed to support hepatocyte differentiation in a defined 3D culture environment. The perfused decellularized matrix allows for perfusion through intact vascular network whereas other decelllularization technologies disrupt the vascular network and extracellular matrix thus not allowing for perfusion.

A reference hESC line, H9 (Madison, WI, http://www.wicell.org) can be cultured and maintained as described in protocols from the provider. Induction of hESC into definitive endoderm (DE) is initiated under conditions without serum in RPMI 1,640 medium (Invitrogen, Carlsbad, CA, http://www.invitrogen.com) supplemented with 100 ng/mL of Activin A (R&D Systems Inc., Minneapolis, http://www.rndsystems.com), 2 mM L-glutamine, and 1% antibiotic-antimycotic for 48 hours. Then the same medium is supplemented with 1xB27 supplement (Invitrogen) and 0.5 mM sodium butyrate for another 3-6 days. The DE cells are then split with trypsin and reseeded at a ratio of 1:1-2 on collagen I-coated plates for hepatic differentiation with the culture medium, supplemented with FGF-4 (20 ng/mL), HGF (20 ng/mL), BMP2, and BMP4 (10 ng/mL each) (R & D Systems). The next day the medium is refreshed with the same medium plus 0.5% dimethyl sulfoxide (DMSO) for 10-14 days. As an alternative, the DE cells are differentiated directly, without splitting, using the same medium mentioned above. Then the cells are further differentiated and maintained in hepatocyte culture medium supplemented with SingleQuots (Lonza, Walkersville, MD, http://www.lonza.com), plus 2-5% fetal bovine serum (FBS), FGF-4 (20 ng/mL), HGF (20 ng/mL), Oncostatin M (50 ng/mL) (R & D Systems), 100 nM Dexamethasone, and 0.5% DMSO until use.

Alternatively, differentiation of MSCs is initiated with a demethylation step using 5'-azacytidine and continued in a specified culture medium that was originally designed to maintain hepatocyte functions of primary human hepatocytes in serum-free long-term culture (Runge et al., BBRC, 209:46 (2000)). When following this protocol, MSCs exhibit features typical of hepatocytes after another 2-3 weeks of culture.

The presence of a functional vascular bed conduit in a decellularized matrix, such as liver matrix, allows for control of stem cell engraftment and characterization of metabolic function *in vitro.* For example, cells are introduced via portal vein perfusion recirculation into liver matrix. Cells may be introduced one or more times, e.g., with 10 minute intervals between each infusion. Cell viability and distribution in the parenchyma may be determined. Engraftment efficiency is determined. In addition, the presence of bile ducts in the decellularized liver matrix, allows for the seeding and control of stem cells engraftment. After seeding, the recellularized liver grafts may be transferred into a specially designed perfusion chamber for *in vitro* culture. The perfusion chamber has two hermetically sealed silicon sheets, forming a pouch filled with culture medium; this design avoids rigid surfaces, preventing development of pressure spots, while enabling sterile culture of the recellularized grafts up to 2 weeks *in vitro.*

The recellularized graft is continuously perfused. Cell viability is maintained during culture, and quantification of TUNEL-positive cells may be conducted, e.g., to determine cells that are apoptotic. The functional characteristics of engrafted cells in the decellularized matrix may be assessed via immunostaining of UDP glucuronosyltransferase 1 family, polypeptide A1 (Ugt1a), a sensitive enzyme with a short half-life (e.g., about 50 minutes) whose presence indicated hepatocyte viability and function, glucose-6-phosphatase, catalytic subunit (G6pc) and albumin. The level of immunostaining for these markers in engrafted hepatocytes is similar to that in normal livers.

Hepatocyte function may be assessed by immunocytochemical detection of CYP1A1 and CYP3A4 proteins, by the periodic acid Schiff (PAS) stain to prove glycogen synthesis and by expression of the hepatocyte markers phosphoenolpyruvate carboxykinase (PCK) and glutamine synthetase (GS) on the protein level using western blot analyses.

To assess the metabolic activity of engrafted hepatocytes, hepatocyte albumin production and urea synthesis may be quantified. Analysis of the expression of drug metabolism enzymes via quantitative RT-PCR may reveal expression levels of drug metabolism enzymes, e.g., *Cyp2c11* (encoding cytochrome P450, subfamily 2, polypeptide 11) *Gstm2* (glutathione S-transferase mu 2), *Ugt1a1* (encoding UDP glucuronosyltransferase-1 family, polypeptide A1) and *Cyp1a1* (encoding cytochrome P450, family-1, subfamily a, polypeptide 1) may be expressed in the recellularized liver at similar levels to those in normal liver. *Adh1* (encoding alcohol dehydrogenase-1) and *Cyp3a18* (encoding cytochrome P450, family 3, subfamily a, polypeptide 18) expression levels may also be determined.

### Controlled System for Decellularizing and/or Recellularizing An Organ or Tissue

A system (e.g., a bioreactor) for decellularizing and/or recellularizing an organ or tissue generally includes at least one cannulation device for cannulating an organ or tissue, a perfusion apparatus for perfusing the organ or tissue through the cannula(s), and means (e.g., a containment system) to maintain a sterile environment for the organ or tissue. Cannulation and perfusion are well-known techniques in the art. A cannulation device generally includes size-appropriate hollow tubing for introducing into a vessel, duct, and/or cavity of an organ or tissue. Typically, one or more vessels, ducts, and/or cavities are cannulated in an organ. A perfusion apparatus can include a holding container for the liquid (e.g., a cellular disruption medium) and a mechanism for moving the liquid through the organ (e.g., a pump, air pressure, gravity) via the one or more cannulae. The sterility of an organ or tissue during decellularization and/or recellularization can be maintained using a variety of techniques known in the art such as controlling and filtering the air flow and/or perfusing with, for example, antibiotics, anti-fungals or other anti-microbials to prevent the growth of unwanted microorganisms.

A system to decellularize and recellularize organ or tissues as described herein can possess the ability to monitor certain perfusion characteristics (e.g., pressure, volume, flow pattern, temperature, gases, pH), mechanical forces (e.g., ventricular wall motion and stress), and electrical stimulation (e.g., pacing). As the coronary vascular bed changes over the course of decellularization and recellularization (e.g., vascular resistance, volume), a pressure-regulated perfusion apparatus is advantageous to avoid large fluctuations. The effectiveness of perfusion can be evaluated in the effluent and in tissue sections. Perfusion volume, flow pattern, temperature, partial O₂ and CO₂ pressures and pH can be monitored using standard methods.

Sensors can be used to monitor the system (e.g., bioreactor) and/or the organ or tissue. Sonomicromentry, micromanometry, and/or conductance measurements can be used to acquire pressure-volume or preload recruitable stroke work information relative to myocardial wall motion and performance. For example, sensors can be used to monitor the pressure of a liquid moving through a cannulated organ or tissue; the ambient temperature in the system and/or the temperature of the organ or tissue; the pH and/or the rate of flow of a liquid moving through the cannulated organ or tissue; and/or the biological activity of a recellularizing organ or tissue. In addition to having sensors for monitoring such features, a system for decellularizing and/or recellularizing an organ or tissue also can include means for maintaining or adjusting such features. Means for maintaining or adjusting such features can include components such as a thermometer, a thermostat, electrodes, pressure sensors, overflow valves, valves for changing the rate of flow of a liquid, valves for opening and closing fluid connections to solutions used for changing the pH of a solution, a balloon, an external pacemaker, and/or a compliance chamber. To help ensure stable conditions (e.g., temperature), the chambers, reservoirs and tubings can be water-jacketed.

It can be advantageous during recellularization to place a mechanical load on the organ and the cells attached thereto. As an example, a balloon inserted into the left ventricle via the left atrium can be used to place mechanical stress on a heart. A piston pump that allows adjustment of volume and rate can be connected to the balloon to simulate left ventricular wall motion and stress. To monitor wall motion and stress, left ventricular wall motion and pressure can be measured using micromanometry and/or sonomicrometry. In some embodiments, an external pacemaker can be connected to a piston pump to provide synchronized stimulation with each deflation of the ventricular balloon (which is equivalent to the systole). Peripheral ECG can be recorded from the heart surface to allow for the adjustment of pacing voltage, the monitoring of deand repolarization, and to provide a simplified surface map of the recellularizing or recellularized heart.

Mechanical ventricular distention can also be achieved by attaching a peristaltic pump to a canula inserted into the left ventricle through the left atrium. Similar to the procedure described above involving a balloon, ventricular distention achieved by periodic fluid movement (e.g., pulsatile flow) through the canula can be synchronized with electrical stimulation.

Using the methods and materials disclosed herein, a mammalian heart can be decellularized and recellularized and, when maintained under the appropriate conditions, a functional heart that undergoes contractile function and responds to pacing stimuli and/or pharmacologic agents can be generated.

A system for generating an organ or tissue may be controlled by a computer-readable storage medium in combination with a programmable processor (e.g., a computer-readable storage medium as used herein has instructions stored thereon for causing a programmable processor to perform particular steps). For example, such a storage medium, in combination with a programmable processor, may receive and process information from one or more of the sensors. Such a storage medium in conjunction with a programmable processor also can transmit information and instructions back to the bioreactor and/or the organ or tissue.

An organ or tissue undergoing recellularization may be monitored for biological activity. The biological activity can be that of the organ or tissue itself such as for cardiac tissue, electrical activity, mechanical activity, mechanical pressure, contractility, and/or wall stress of the organ or tissue. In addition, the biological activity of the cells attached to the organ or tissue may be monitored, for example, for ion transport/exchange activity, cell division, and/or cell viability. See, for example, Laboratory Textbook of Anatomy and Physiology (2001, Wood, Prentice Hall) and Current Protocols in Cell Biology (2001, Bonifacino et al., Eds, John Wiley & Sons). As discussed above, it may be useful to simulate an active load on an organ during recellularization. A computer-readable storage medium of the invention, in combination with a programmable processor, may be used to coordinate the components necessary to monitor and maintain an active load on an organ or tissue.

In one embodiment, the weight of an organ or tissue may be entered into a computer-readable storage medium as described herein, which, in combination with a programmable processor, can calculate exposure times and perfusion pressures for that particular organ or tissue. Such a storage medium may record preload and afterload (the pressure before and after perfusion, respectively) and the rate of flow. In this embodiment, for example, a computer-readable storage medium in combination with a programmable processor can adjust the perfusion pressure, the direction of perfusion, and/or the type of perfusion solution via one or more pumps and/or valve controls.

The invention will be further described in the following examples, which do not limit the scope of the invention described in the claims.

### Example 1

### Comparison of Perfusion vs. Immersion

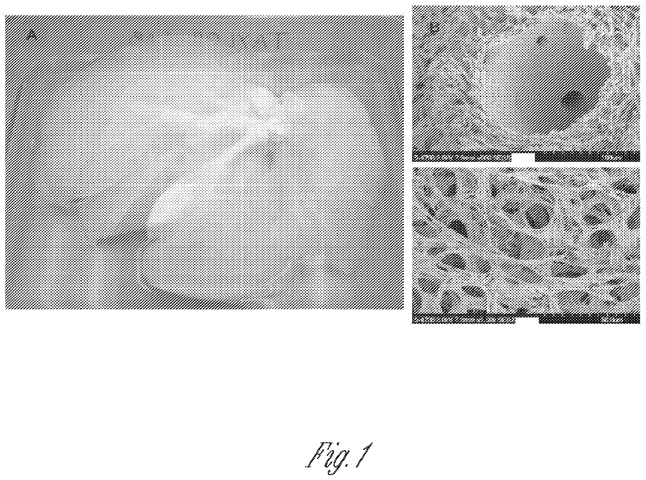

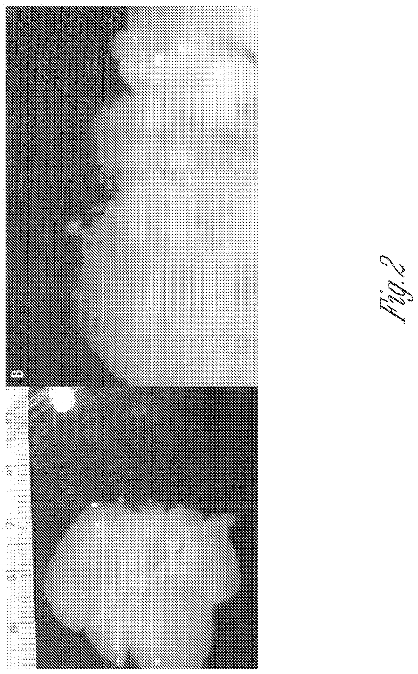

Figure 1A shows a photograph of a porcine liver that was perfusion decellularized, and Figure 1B and 1C show SEM of a vessel and the parenchymal matrix, respectively, of the perfusion decellularized porcine liver. These photographs show the vascular conduits and the matrix integrity of a perfusion decellularized organ. On the other hand, Figure 2 shows a gross view of an immersion decellularized rat liver, in which fraying of the matrix can be seen at both low (left) and high (right) magnification.

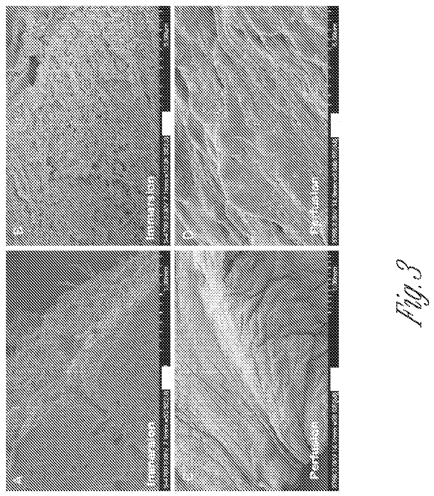

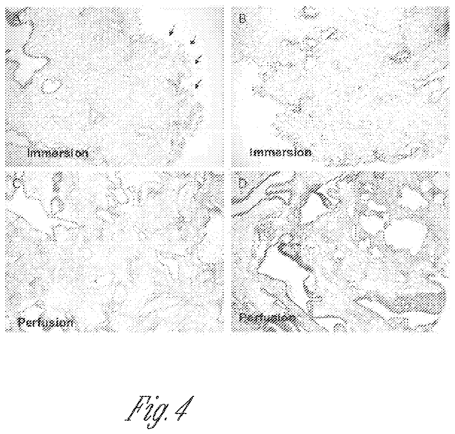

Figure 3 shows SEM of immersion decellularized rat liver (A and B) and perfusion decellularized rat liver (C and D). These results clearly indicate that immersion decellularization significantly compromised the organ capsule (Glisson's capsule), while perfusion decellularization retained the capsule. In addition, Figure 4 shows histology of immersion decellularized liver (A, H&E staining; B, Trichrome staining) and perfusion decellularized liver (C, H&E staining; D, Trichrome staining). The immersion decellularized rat liver did not retain cells or dye upon injection.

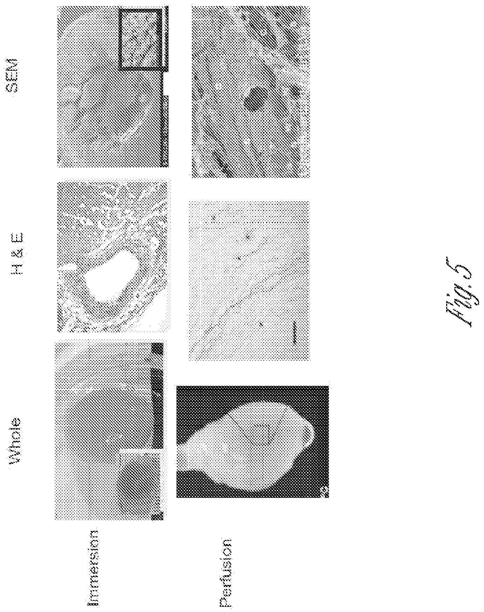

Figure 5 shows a comparison between immersion decellularization (top row) and perfusion decellularization (bottom row) of a rat heart. The photographs in the left column show the whole organ. As can be seen from the two photographs, the perfusion decellularized organ (bottom left) is much more translucent than the immersion decellularized organ (top left), which retains the iron-rich "brown-red" color of cadaveric muscle tissue and appears to still contain cells. The photographs in the middle column show the H&E staining pattern of the decellularized tissues. The staining shows that a number of cells, both within the parenchyma and in the walls of the vasculature, remain following immersion decellularization (top middle), while virtually every cell and also the cellular debris is removed following perfusion decellularization (bottom middle) even as patent vascular conduits are evident. In addition, the scanning electron micrographs in the right column show that there is a significant difference in the ultrastructure of the matrix following immersion (top right) vs. perfusion (bottom right) decellularization. Again, complete retention of cellular components throughout the cross section of the myocardium was observed in all the walls of the immersion-decellularized heart, but almost a complete loss of these cellular components was observed in the perfusion-decellularized heart along with the retention of spatial and architectural features of the intact myocardium including vascular conduits. For example, the perfusion-decellularized matrix retained the architectural features within the matrix including weaves (w), coils (c) and struts (s) despite the complete loss of cells.

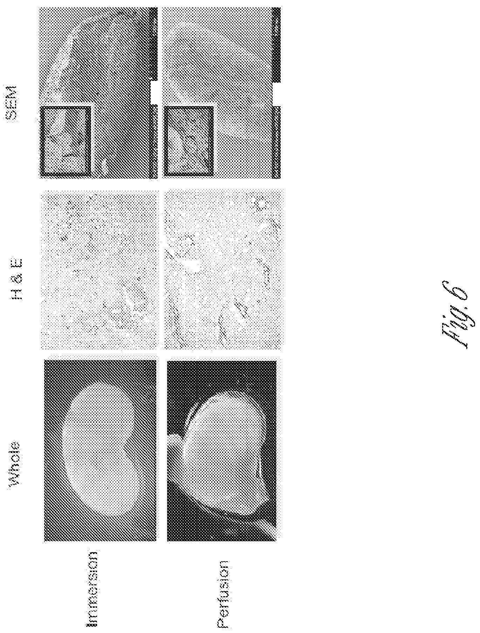

Figure 6 shows the same comparisons (immersion decellularization (top row) vs. perfusion decellularization (bottom row)) using rat kidney. Unlike heart, the immersion-decellularized whole kidney (top left) looks grossly similar to the perfusion-decellularized whole kidney (bottom left) in that both are fairly translucent. However, in the perfusion-decellularized kidney, the network of vascular conduits within the perfusion-decellularized organ is more obvious and a greater degree of branching can be visualized than in the immersion-decellularied construct. Furthermore, the perfusion-decellularized kidney retains an intact organ capsule, is surrounded by mesentery, and, as shown, can be decellularized along with the attached adrenal gland. The photographs in the center column show the H&E staining pattern of the two tissues. The staining shows that cellular components and/or debris and possibly even intact nuclei (purple stain) remain following immersion-decelluarization (top center), while virtually every cell and/or all cellular debris is removed following perfusion-decellularization (bottom center). Likewise, the SEM photographs demonstrate that the immersion-decellularized kidney matrix (top right) suffered much more damage than did the perfusion-decellularized kidney matrix (bottom right). In the immersion-decellularized kidney, the organ capsule is missing or damaged such that surface "holes" or fraying of the matrix are obvious, whereas, in the perfusion decellularized organ, the capsule is intact.

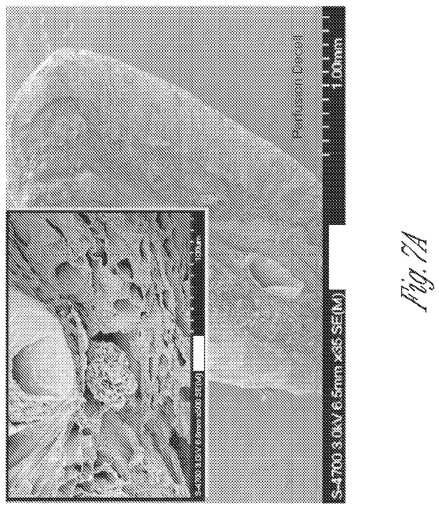

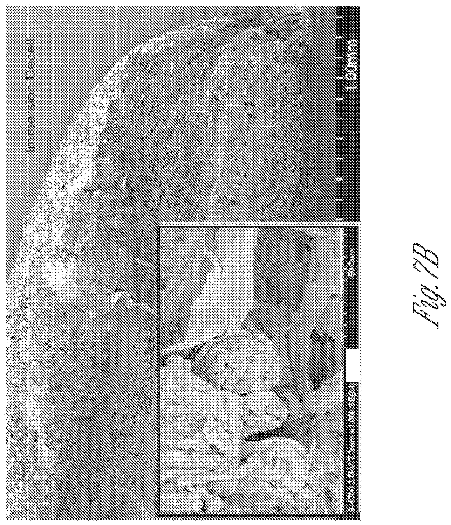

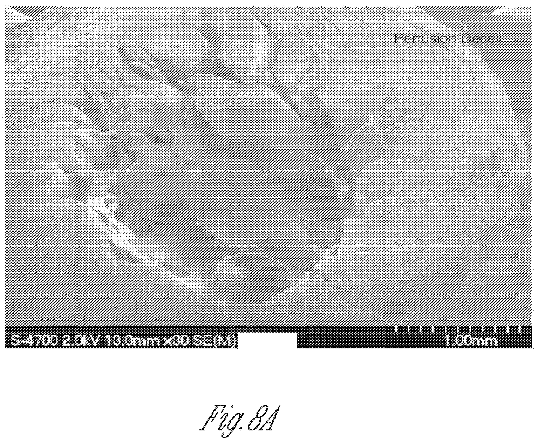

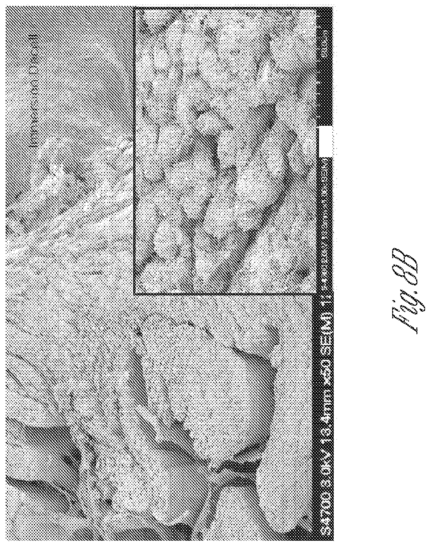

Figure 7 shows SEM photographs of decellularized kidney. Figure 7A shows a perfusion-decellularized kidney, while Figure 7B shows an immersion-decellularized kidney. Figure 8A shows a SEM photograph of a perfusion-decellularized heart, while Figure 8B shows a SEM photograph of an immersion-decellularized heart. These images further demonstrate the damage that immersion-decellularization caused to the ultrastructure of the organ, and the viability of the matrix following perfusion-decellularization.

### Example 2

Prior to recellularization of ECM or during culturing in ECM, cardiac phenotype may be assessed using the following markers: c-MHC, c-TNT, sarcomere formation, and myofibril organization, as well as spontaneous contractions. During the induction of differentiation, the time-course for mRNA levels of cardiac-specific genes may be monitored. Genes coding for a cardiac-specific transcription factor Nkx2.5, a cardiac structure protein α-myosin heavy chain (α-MHC), and a cardiac-specific peptide hormone atrial natriuretic factor (ANF), may be followed.

In addition, the electrical potentials of cells may be assessed by the spontaneous beating of cells showing extracellular field potentials, and the frequency and timing of beating. To quantitatively assess the number of cardiomyocytes in the total population, intracellular FACS analyses may be performed using an anti-c-MHC antibody. Intra-cellular FACS analysis with an antibody against cardiac-troponin T (c-TNT), a specific protein for cardiac muscle cells, may also be performed. Other markers which may be detected are a zinc finger transcription factor, GATA4, a lateral mesoderm marker, vascular endothelial growth factor (VEGF) receptor-2 (Flk-1), its ligand, VEGF, an intrinsic histone acetyltransferases, p300, a member of class II HDAC, HDAC4, and a stem cell marker, Oct3/4.

Recellularized (e.g., with 50 x 10⁶ partially committed cells optionally in combination with fibrocytes, endothelial cells and/or smooth muscle cells) scaffolds may be mounted in a perfusable bioreactor, e.g., one that simulates cardiac physiology including pulsatile left ventricular distension with gradually increasing preload and afterload, pulsatile coronary flow, and electric stimulation under sterile cardiac tissue culture conditions (5% CO₂, 60% H₂O, 37°C). Perfused organ culture is maintained for one to four weeks. Pressures, flows and EKG may be recorded for 30 seconds every 15 minutes throughout the entire culture period.

At later time points, a more in-depth functional assessment may be performed including insertion of a pressure probe into the left ventricle to record left ventricular pressure (LVP) as the stimulation frequency is gradually increased from 0.1 Hz to 10 Hz and pharmacological stimulation is conducted with phenylephrine (PE). The recellularized heart may show contractile response to single paces with spontaneous contractions following the paced contractions with corresponding increases in LVP. Similar to the stimulated contractions, spontaneous depolarizations causes a corresponding increase in LVP and a recordable QRS complex possibly indicating the formation of a developing stable conduction pattern.

### Example 3

One use for a recellularized matrix of the invention is to screen drugs. For example, to achieve a pharmaceutical testing system for drug metabolism and toxicity, human hepatocytes may be seeded into a perfusion decellularized liver matrix. The construct may also be seeded with sinusoidal endothelial cells if desired. The liver construct may then be perfused under normal physiological conditions with cell culture media designed to maintain functional hepatocyctes such as, but not limited to, Williams' Medium E. The liver constructs are then exposed to various drugs for a specified period of time. The constructs are then assayed for various liver functions including albumin, urea, G6PDH, and various CYP functions including CYP1A2, CYP3A4, etc. to determine the overall effect on the liver construct and provide metabolic and toxicity information. Similar studies may be performed using other organs including but not limited to human seeded constructs for heart and kidney to look at organ specific drug toxicity.

### Example 4

Another use for a recellularized matrix is to provide a source for cells of a certain type, e.g., differentiated cells or functionally mature cells. In one embodiment, partially differentiated cardiac ES or iPS cells are cultured on a perfusion decellularized cardiac matrix (ECM scaffold) for a time, e.g., about 2 to about 80 days, and under conditions so as to yield cardiac cells, e.g., a substantially pure population such as one having greater than about 50% atrial cardiomyocytes when cultured on atrial cardiac ECM, greater than about 50% ventricular cardiomycytes when cultured on ventricular ECM, and/or greater than about 50% nodal cardiomyocytes when cultured on nodal ECM, which cardiomycytes are identified by their functional action potential shapes and/or action potential durations, e.g., an atrial action potential diplaying typical triangle-like action potentials compared to the prominent plateau displayed by ventricular cardiomyocytes. This classification is based on the properties of the action potential as measured by the maximum rate of rise of the action potential (dV/dtmax), the action potential duration (APD), action potential amplitude (APA), and prominence of phase-4 depolarization. Nodal-like action potentials were characterized by prominent phase-4 depolarization, slow upstroke (dV/dtmax), and a smaller APA. Ventricular action potentials may be distinguished by the presence of a significant plateau phase of the action potential resulting in a significantly longer duration compared to the more triangular shaped embryonic-atrial action potentials. These electrophysiology properties are quite distinct from neonatal and adult cardiac muscle. In particular, the embryonic action potentials are characterized by more depolarized maximum diastolic potentials (MDP) and "slow" type action potentials based on low dV/dtmax (about 5 to about 30 V/sec). These cells are then isolated from the construct through the digestion of the matrix with matrix digesting enzyme mixtures containing collagenases either perfused through the heart or cutting up the heart and placing the resulting portions into culture for about 1 hour to about24 hours.

In one embodiment, partially differentiated hepatocytes are seeded in low numbers and cultured on a perfusion decellularized liver scaffold for a time, e.g., about 2 to about 80 days, and under conditions so to yield hepatocyte expansion followed by functional maturation once confluent, as defined by markers, e.g., including but limited to albumin and urea expression. Functional hepatocytes are then isolated from the liver construct through digestion of the matrix with various matrix digesting enzymes such as collagenases either through perfusion or static culture for about 2 hours to about 24 hours.

While in the foregoing specification, this invention has been described in relation to certain preferred embodiments thereof, and many details have been set forth for purposes of illustration, it will be apparent to those skilled in the art that the invention is susceptible to additional embodiments and that certain of the details herein may be varied considerably without departing from the basic principles of the invention.

## Claims

1. A method to prepare a perfusion based *in vitro* 3D cell culture system, comprising:
selecting a perfusion decellularized matrix of a liver or pancreas having an intact vascular network and a population of stem cells capable of differentiation to a cell type present in a native liver or pancreas; and
perfusing the population of cells into the perfusion decellularized matrix under conditions that allow for seeding of the perfusion decellularized matrix and for differentiation and functional maturation of the stem cells in the population to cell types present in the liver or pancreas prior to decellularization, wherein the conditions also include perfusing the matrix with medium that contains activators or inhibitors of differentiation pathways selected to provide for liver cell- or pancreas cell-specific differentiation.

2. The method of claim 1 wherein the matrix is a liver matrix.

3. The method of claim 1 or 2 wherein the cells are iPS cells.

4. The method of claim 1 wherein the matrix is a pancreas matrix.

5. The method of claim 2 wherein the cells are capable of differentiation into functional hepatocytes and optionally express albumin, express HepPar1, and/or deposit glycogen or optionally release albumin and urea.

6. The method of claim 4 wherein the cells are capable of differentiation into functional beta-cells and optionally release insulin in response to glucose stimulation.

7. The method of any one of claims 1 to 6 wherein the population comprises a plurality of different cell types.

8. The method of any one of claims 1 to 7 wherein the cells and the perfusion decellularized organ or tissue are allogeneic or xenogeneic.

9. The method of claim 8 wherein the cells and the perfusion decellularized organ or tissue are xenogeneic and wherein the liver extracellular matrix is derived from a small animal, e.g. a nonhuman mammal.

10. The method of claim 1 wherein the medium that contains activators or inhibitors includes one or more of Activin A, bFGF, retinoic acid (RA), Wnt3A, KGF, KAAD-cyclopamine, Noggin, or nicotinamide for pancreatic differentiation and functional maturation.

11. The method of claim 1 wherein the medium that contains activators or inhibitors includes one or more of Activin A, BMP, FGF, dexamethasone, IL6, HGF, Oncostatin M, or EGF for liver differentiation and functional maturation.

12. The method of claim 1 wherein the cells are seeded into the interstitial space or matrix outside of the vascular conduits.

## Patentansprüche

1. Verfahren zur Herstellung eines Perfusions-basierten *in vitro*-3D-Zellkultursystems, bei dem man:
eine durch Perfusion dezellularisierte Matrix von einer Leber oder einer Pankreas mit einem intakten vaskulären Netzwerk und eine Population von Stammzellen auswählt, die in der Lage sind, in einen Zelltyp zu differenzieren, welcher in einer nativen Leber oder Pankreas vorkommt; und
die Zellpopulation in die durch Perfusion dezellularisierte Matrix unter Bedingungen einschwemmt, die ein Ansäen der durch Perfusion dezellularisierten Matrix und eine Differenzierung und funktionale Reifung der Stammzellen in der Population zu Zelltypen ermöglicht, die vor der Dezellularisierung in der Leber oder in der Pankreas vorhanden waren, wobei die Bedingungen auch die Perfusion der Matrix mit einem Medium umfasst, das Aktivatoren oder Inhibitoren von Differenzierungswegen enthält, die so ausgewählt sind, dass eine für Leberzellen oder Pankreaszellen spezifische Differenzierung erfolgt.

2. Verfahren gemäß Anspruch 1, wobei die Matrix eine Leber-Matrix ist.

3. Verfahren gemäß Anspruch 1 oder 2, wobei die Zellen iPS-Zellen sind.

4. Verfahren gemäß Anspruch 1, wobei die Matrix eine Pankreas-Matrix ist.

5. Verfahren gemäß Anspruch 2, wobei die Zellen in der Lage sind, in funktionsfähige Hepatozyten zu differenzieren und wahlweise Albumin zu exprimieren, HepPar1 zu exprimieren und/oder Glykogen zu speichern oder wahlweise Albumin und Urea freizusetzen.

6. Verfahren gemäß Anspruch 4, wobei die Zellen in der Lage sind, in funktionierende Beta-Zellen zu differenzieren und wahlweise Insulin in Reaktion auf eine Glukose-Stimulierung freizusetzen.

7. Verfahren gemäß einem der Ansprüche 1 bis 6, wobei die Population eine Vielzahl von verschiedenen Zelltypen umfasst.

8. Verfahren gemäß einem der Ansprüche 1 bis 7, wobei die Zellen und das durch Perfusion dezellularisierte Organ oder Gewebe allogen oder xenogen sind/ist.

9. Verfahren gemäß Anspruch 8, wobei die Zellen und das durch Perfusion dezellularisierte Organ oder Gewebe xenogen sind/ist, und wobei die extrazelluläre Matrix der Leber von einem kleinen Tier erhalten wurde, beispielsweise einem nicht-menschlichen Säugetier.

10. Verfahren gemäß Anspruch 1, wobei das die Aktivatoren oder Inhibitoren enthaltende Medium eine oder mehrere der folgenden Substanzen enthält: Activin A, bFGF, Retinolsäure (RA), Wnt3A, KGF, KAAD-Cyclopamin, Noggin oder Nikotinamid zur pankreatischen Differenzierung und funktionalen Reifung.

11. Verfahren gemäß Anspruch 1, wobei das die Aktivatoren oder Inhibitoren enthaltende Medium eine oder mehrere der folgenden Substanzen enthält: Activin A, BMP, FGF, Dexamethason, IL6, HGF, Onkostatin M oder EGF zur Differenzierung und funktionalen Reifung der Leber.

12. Verfahren gemäß Anspruch 1, wobei die Zellen in die Zwischenräume oder in die Matrix außerhalb der vaskulären Gefäße ausgesät werden.

## Revendications

1. Procédé de préparation d'un système tridimensionnel de culture cellulaire *in vitro* à base de perfusion, comportant les étapes suivantes :
- sélectionner une matrice de foie ou de pancréas décellularisée par perfusion, qui présente un réseau vasculaire intact, et une population de cellules souches capables de se différentier en cellules d'un type présent dans un foie ou un pancréas natif ;
- et faire perfuser la population de cellules dans la matrice décellularisée par perfusion, dans des conditions qui permettent l'ensemencement de la matrice décellularisée par perfusion et la différentiation et la maturation fonctionnelle des cellules souches de la population en cellules de types présents dans le foie ou le pancréas avant décellularisation, lesquelles conditions incluent aussi le fait de faire perfuser dans la matrice un milieu qui contient des activateurs ou des inhibiteurs de voies de différentiation sélectionnés pour assurer une différentiation spécifique de cellules du foie ou spécifique de cellules du pancréas.

2. Procédé conforme à la revendication 1, dans lequel la matrice est une matrice de foie.

3. Procédé conforme à la revendication 1 ou 2, dans lequel les cellules sont des cellules souches pluripotentes induites (iPS).

4. Procédé conforme à la revendication 1, dans lequel la matrice est une matrice de pancréas.

5. Procédé conforme à la revendication 2, dans lequel les cellules sont capables de se différentier en hépatocytes fonctionnels et, en option, expriment de l'albumine, expriment de l'HepPar1 et/ou accumulent du glycogène ou, en option, libèrent de l'albumine et de l'urée.

6. Procédé conforme à la revendication 4, dans lequel les cellules sont capables de se différentier en cellules bêta fonctionnelles et, en option, libèrent de l'insuline en réponse à une stimulation par du glucose.

7. Procédé conforme à l'une des revendications 1 à 6, dans lequel la population de cellules comprend des cellules de plusieurs types différents.

8. Procédé conforme à l'une des revendications 1 à 7, dans lequel les cellules et l'organe ou le tissu décellularisé par perfusion sont allogéniques ou xénogéniques.

9. Procédé conforme à la revendication 8, dans lequel les cellules et l'organe ou le tissu décellularisé par perfusion sont xénogéniques et dans lequel la matrice extracellulaire de foie provient d'un petit animal, par exemple d'un mammifère non humain.

10. Procédé conforme à la revendication 1, dans lequel le milieu qui contient des activateurs ou des inhibiteurs contient l'un ou plusieurs des suivants : activine A, bFGF, acide rétinoïque (RA), Wnt3A, KGF, KAAD-cyclopamine, noggine et nicotinamide, pour une différentiation et une maturation fonctionnelle en cellules pancréatiques.

11. Procédé conforme à la revendication 1, dans lequel le milieu qui contient des activateurs ou des inhibiteurs contient l'un ou plusieurs des suivants : activine A, BMP, FGF, dexaméthasone, IL-6, HGF, oncostatine M et EGF, pour une différentiation et une maturation fonctionnelle en cellules hépatiques.

12. Procédé conforme à la revendication 1, dans lequel les cellules sont semées dans l'espace interstitiel ou la matrice, hors des conduits vasculaires.
